# EUROPÄISCHE PATENTANMELDUNG

(11) **EP 1 655 053 A1**
(43) Veröffentlichungstag der Anmeldung: **10.05.2006**
(21) Anmeldenummer: 05024188.4
(22) Anmeldetag: 07.11.2005
(51) Int. Cl.: A61N 1/04, A61B 5/04, A61F 13/02

(54) **Befestigungsmittel für therapeutische und/oder diagnostische Medien**

(30) Priorität: 08.11.2004 AT 18612004
(71) Anmelder: HARTL, Friedrich, 1100 Wien (AT)
(72) Erfinder: HARTL, Friedrich, 1100 Wien (AT)
(74) Vertreter: Puchberger, Peter

(57) **Zusammenfassung**

Befestigungsmittel für therapeutische und/oder diagnostische Medien, wie zum Beispiel therapeutische und/oder diagnostische Elektroden, Kälte- beziehungsweise Wärmeträger, Moorpackungen und dergleichen, an Extremitäten oder Körperteilen von Tieren oder Menschen, dadurch gekennzeichnet, dass die Befestigungsmittel (11) ein oder mehrere Streifen einer Stretchfolie (5) sind.

## Beschreibung

Die Erfindung betrifft ein Befestigungsmittel für therapeutische und/oder diagnostische Medien, wie zum Beispiel therapeutische und/oder diagnostische Elektroden, Kältebeziehungsweise Wärmeträger, Moorpackungen und dergleichen, an Extremitäten oder Körperteilen von Tieren oder Menschen, und ein Verfahren für die Befestigung derartiger Medien.

Bekannte Befestigungsmittel für eine Reihe von therapeutischen und/oder diagnostischen Medien erfüllen zwar hinsichtlich der Verbindung vom Medium mit der betreffenden Körperstelle ihren Zweck, sind aber mit einer Reihe von Nachteilen behaftet. So zum Beispiel müssen bisher verwendete Gummigurte oder Kurzzugbinden zum Anbringen therapeutischer Elektroden zeit- und kostenintensiv hygienisch vorbereitet werden, wie etwa durch thermische Desinfektion oder Wischdesinfektion mit chemischen Desinfektionsmitteln. Des weiteren können Gummigurte oder dergleichen Allergien gegen Latex, Gummibestandteile oder ähnlichem hervorrufen.

Bei der Befestigung von EKG-Elektroden mittels bekannter selbstklebender Kontakte verbleiben oft Rückstände des Kontakt-Klebers am Körper, wobei die Anwendung solcher medizinischer Einmalprodukte bei Verwendung im größeren Maßstab schnell sehr teuer wird. Bei der Benutzung von Unterdruck-Saugelektroden an behaarten Körperstellen, fallen diese oftmals während der Aufzeichnung des EKG-Signals ab, was eine Wiederholung der Untersuchung erforderlich macht.

Befestigungsmittel für Elektroden, z. B. für die Elektrotherapie müssen darüber hinaus möglichst mit gleichem Anpressdruck an ihrer gesamten Fläche mit der Haut kraftschlüssig verbunden werden können.

Bisher in der Elektrotherapie verwendete Befestigungsmittel waren im Stand der Technik ausschließlich
a) Gummibänder von ca. 3 cm Breite,
b) Kurzzugbinden aus elastischen Textilien oder
c) Klettbänder aus elastischen Textilien.

In Bezug auf Gummibänder von ca. 3 cm Breite ergibt sich der Nachteil, dass infolge der im Vergleich zur Elektrodengröße von meist 100, 200 oder 300 cm² schmalen Breite der Gummibänder es zu einem äußerst unterschiedlichen Anpressdruck in den einzelnen Zonen der Elektroden kommt.

Daher waren in der Vergangenheit immer wieder Verätzungen der Haut bedingt durch einen zu hohen Übergangswiderstand zwischen Elektrode und Haut an einzelnen Stellen der Elektrode mit zu geringem Anpressdruck die Folge.

Bei den bisher verwendeten textilen elastischen Bandagen ändert sich durch die zunehmende Feuchtigkeitsdurchtränkung der Bandage, die damit befestigte Elektrode ist ebenfalls feuchtigkeitsgesättigt, kontinuierlich der Elastizitätskoeffizient, sodass es zu einem Abnehmen des Anpressdruckes mit längerer Verweildauer der Bandage kommt, was bei ungleichmäßiger Durchfeuchtung und einem unregelmäßigen Anpressdruck bis hin zu einem Abheben der Elektroden führen kann, woraus sich die Gefahr von Verätzungen der Haut ergibt.

Weiters ermöglichen textile elastische Befestigungsbandagen nicht die Sicht auf die mit Ihnen an den Patienten angewickelte Elektrode. Allfällige Falten sind dadurch nicht sichtbar und können nicht korrigiert werden. Entstehende Falten können ebenfalls zu Verätzungen führen.

Bei der Verwendung von Kettbänder aus elastischen Textilien verlieren diese nach dem Waschen in der Waschmaschine bei 85 Grad zur Desinfektion nach wenigen Durchgängen die Elastizität. Ein hygienisch einwandfreies Vorgehen wie das Waschen nach jedem Patienten, ist daher aus wirtschaftlichen Gründen nicht möglich, da diese Klettbänder sehr teuer sind. Mit Alkohol sind sie aufgrund der rauen Oberfläche ebenfalls nicht zufriedenstellend zu desinfizieren.

Beim Platzieren von Verbänden, Moor- oder Heilpackungen, werden oft mehrere Befestigungsmittel benötigt, wie etwa Mullbinden, Pflaster, elastische Stützgewebe, etc., wobei die Anwendung meist aufwändig ist und in manchen Fällen oftmaligen Verbandwechsel erfordert, da die Befestigungsmittel meist durchlässig sind und schnell verschmutzen.

Aufgabe der Erfindung ist es nun ein Befestigungsmittel für therapeutische und/oder diagnostische Medien zu schaffen, welches hygienisch, schnell und einfach Anzubringen und zu Entfernen ist, keine Allergien an der betroffenen Person oder dem betroffenen Tier hervorruft und bei der Anschaffung und Verwendung die Kosten gering hält.

Gelöst wird diese Aufgabe durch die Erfindung in der Art, dass das Befestigungsmittel für die therapeutischen und/oder diagnostischen Medien ein oder mehrere Streifen einer Stretchfolie sind. Da es sich bei diesen Folien um in der Anschaffung billige Einmalprodukte handelt, entfällt die Desinfektion des Befestigungsmittels, was die Hygiene verbessert und Zeit und Kosten spart.

Gemäß einem Merkmal der Erfindung sind diese Folien im wesentlichen aus Polyethylen hergestellt, welches nur aus Kohlenwasserstoff besteht und damit keine der bekannten Allergien gegen Latex, Gummibestandteile oder dergleichen hervorrufen kann.

Ein weiteres Merkmal der Erfindung sieht vor, dass die Bündelstretchfolie eine Dehnspannung beim Zerreißen nach ISO 527-3 Norm von 250% bis 550% aufweist, einen Reibungskoeffizienten von in etwa 0,8 hat und vorzugsweise in einer Breite von etwa 10 cm und einer Dicke von 10 µm bis 60 µm Verwendung findet. Durch diese besonderen Eigenschaften haftet die Folie beim Umwickeln aufgrund ihrer besonderen Glätte mittels der Wirkung von Adhäsionskräften aneinander. Aus diesem Grund benötigt man keine weiteren Befestigungen zum Anbringen der therapeutischen und/oder diagnostischen Medien, da die Folie die entsprechenden Medien sicher an der gewünschten Stelle fixiert.

Weiters wird durch die Anwendung einer Stretchfolie in einer Breite von 100 mm ein gleichmäßiger Anpressdruck der Elektrode gewährleistet.

Der Elastizitätskoeffizient der in der erfindungsgemäßen Befestigung verwendeten Folie wurde so gewählt, dass ein Herabfallen beispielsweise einer Elektrode in Folge einer Ausdehnung durch den Gegendruck der Elektrode verhindert wird, andererseits aber ein Einschnüren der Körperteile an denen die Elektroden befestigt sind verhindert wird. Eine derartige Befestigung bleibt zeitunabhängig konstant.

Die Breite der Folie, die Dicke der Folie und die Festlegung der Glätte wurden so gewählt, dass eine optimale Befestigung erzielbar ist. Schmälere Folien führen zu ungleichmäßigem Anpressdruck der Elektroden mit Verätzungsgefahr, breitere Folien überstehen die verwendeten Elektroden und sind im Handling nicht verwendbar, da aufgrund der sphärischen Körperform Faltenbildungen zu verzeichnen sind.

Die verwendete Glätte und die übrigen Materialeigenschaften verhindern auch das Entwickeln eines kapillaren Feuchtigkeitsfilms zwischen den einzelnen Lagen der Anwicklung der Folie, der zur Verbreitung von Kriechströmen Anlass geben könnte, wodurch unerwünschte Effekte bei der Anwendung von Elektrotherapie bishin zum Auftreten von Verätzungen möglich wären.

Des weiteren umfasst die Erfindung ein Verfahren zur Befestigung von therapeutischen und/oder diagnostischen Medien, wie zum Beispiel therapeutische und/oder diagnostische Elektroden, Kälte- beziehungsweise Wärmeträger, Moorpackungen und dergleichen, an Extremitäten oder Körperteilen von Tieren oder Menschen. Dieses Verfahren ist dadurch gekennzeichnet, dass das therapeutische und/oder diagnostische Medium an einer Extremität oder einem Körperteil eines Tieres oder eines Menschen platziert wird und durch zumindest einmaliges Umwickeln mit Bündelstretchfolie befestigt wird. Gegebenenfalls wird die Befestigung durch die genannte Bündelstretchfolie nach durchgeführter Therapie und/oder Diagnose durch Zerschneiden, anderwärtiges Durchtrennen oder Abwickeln der Folie wieder entfernt.

Weitere vorteilhafte Merkmale der Erfindung sind der Beschreibung, den Ansprüchen und den Zeichnungen zu entnehmen. Nachfolgend wird die Erfindung anhand einiger Ausführungsbeispiele und der Zeichnungen näher beschrieben.

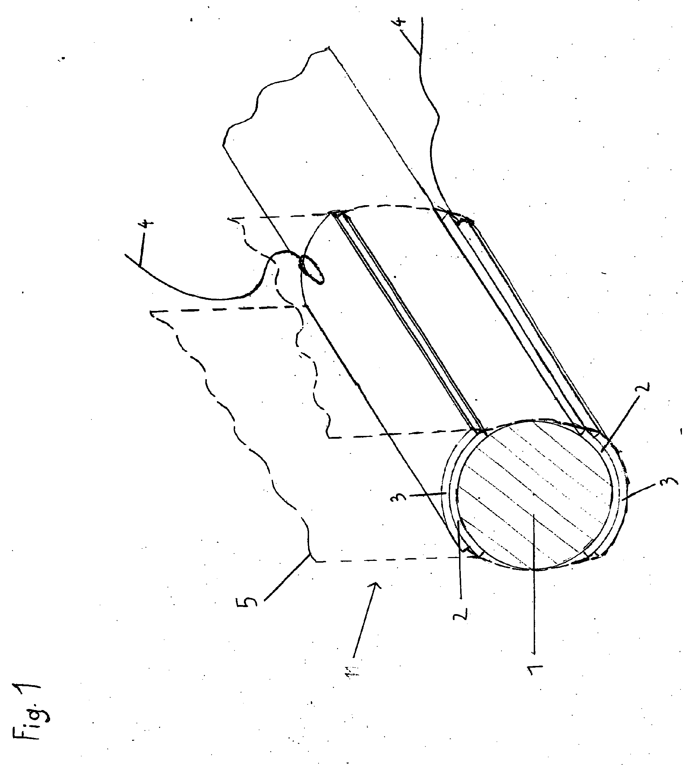

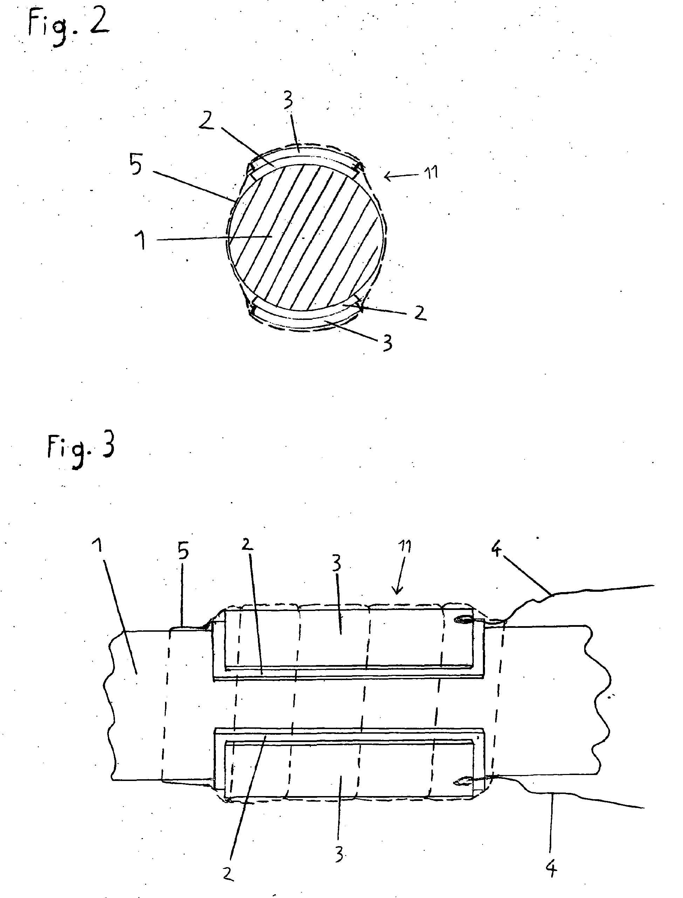

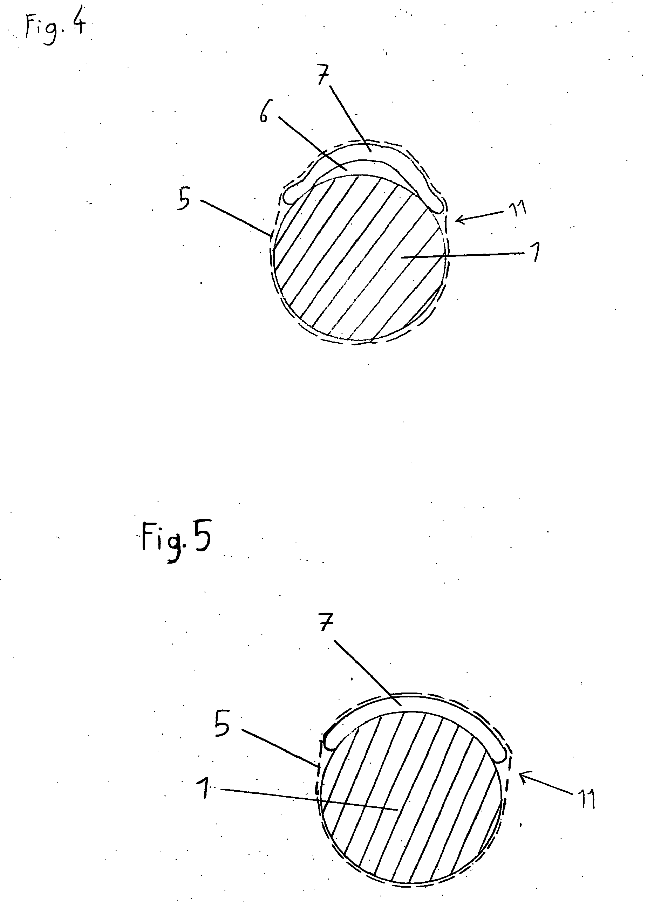

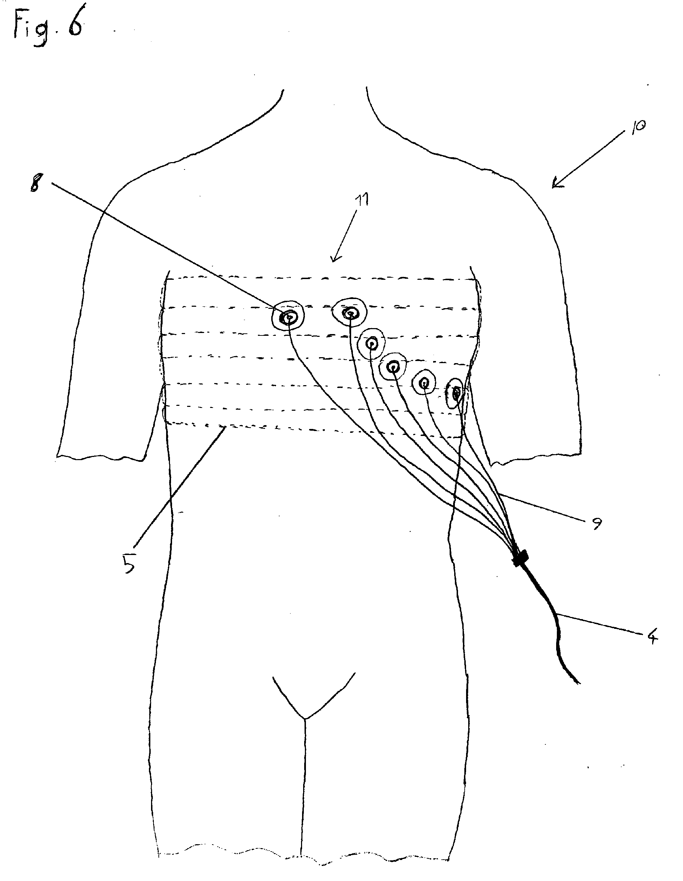

Fig. 1 zeigt eine perspektivische Querschnitts-Detailansicht einer Extremität mit therapeutischer Elektrodenanordnung. Fig. 2 ist eine Querschnittsansicht einer Extremität mit befestigter therapeutischer Elektrodenanordnung. Fig. 3 ist eine Detailansicht einer Extremität mit befestigter therapeutischer Elektrodenanordnung. Fig. 4 zeigt eine Querschnittsansicht einer Extremität mit einer befestigten Moorpackung. Fig. 5 ist eine Querschnittsansicht einer Extremität mit einem befestigten Wärme- bzw. Kälteträger. Fig. 6 zeigt einen Torso mit befestigten EKG-Elektroden.

Die Fig. 1 zeigt eine perspektivische Querschnitts-Detailansicht einer Extremität (1) wie zum Beispiel eines Unterarms mit therapeutischer Elektrodenanordnung. An der Extremität (1) werden zunächst angefeuchtete Zwischenlagen (2) aus Schaumgummimaterial, Frotte oder dergleichen aufgelegt. Durch die mit Wasser getränkten Zwischenlagen (2) wird die Elektrolytbildung, welche bei der Zufuhr therapeutischer Ströme in der Größenordnung von bis zu 60 Volt Spannung und bis zu 60 mA Stromstärke entsteht, unschädlich gemacht und ein gleichmäßiger Übertragungswiderstand für den zugeführten Strom sichergestellt. Auf die angefeuchteten Zwischenlagen (2) werden die Elektroden (3) platziert, welche mittels Stromleitungen (4) an einem Gerät zur Abgabe therapeutischer Ströme (nicht gezeigt) angeschlossen sind. Über diese Extremität (1) mit den angeordneten Elektroden wird nun die Bündelstretchfolie (5) als Befestigungsmittel (11) gewickelt, welche mittels Adhäsion an sich selbst haftet und daher die Anordnung sicher befestigt. Durch mehrmaliges Umwickeln kann die Festigkeit der Befestigung (11) erhöht werden. Beim Wickeln ist der Druck vom Arzt oder der von diesem betrauten Hilfsperson gut dosierbar. Es versteht sich, dass eine Anordnung zur Abnahme von diagnostischen Strömen im Prinzip zu Fig. 1 gleich aufgebaut ist, nur dass die Stromleitungen (4) anstelle an einem Gerät zur Abgabe therapeutischer Ströme, an einem Messgerät wie zum Beispiel einem EKG angeschlossen sind.

In Fig. 2 sieht man einen Querschnitt durch die in Fig. 1 beschriebene Anordnung nach dem Wickeln. Auf der Extremität (1) befinden sich die angefeuchteten Zwischenlagen (2) und darauf die Elektroden (3). Die gesamte Anordnung ist durch die umwickelte Bündelstretchfolie (5) sicher und dennoch angenehm für den Patient befestigt.

Fig. 3 zeigt eine seitliche Detailansicht der Extremität (1) mit den angefeuchteten Zwischenlagen (2) und Elektroden (3) von welchen die Stromleitungen (4) wegführen. Wie hier ersichtlich ist, wird die Anordnung durch mehrmaliges spiralförmiges Umwickeln mit Bündelstretchfolie (5), welche hier strichliert dargestellt ist, befestigt.

In Fig. 4 ist ein Querschnitt durch eine Extremität (1) mit einer aufgebrachten Moorpackung (6). Die aufgetragene Moorpackung (6) wird zusammen mit einem darüber angeordneten Wärme- bzw. Kälteträger (7) ebenfalls durch umwickeln mittels Bündelstretchfolie (5) an der gewünschten Stelle fixiert.

Fig. 5 zeigt einen Querschnitt durch eine Extremität (1), auf welcher ein Wärme- bzw. Kälteträger (7) mittels Bündelstretchfolie (5) befestigt ist. Der Wärme- bzw. Kälteträger (7) kann entweder direkt befestigt werden, oder aber unter einer textilen Zwischenlage am Körper befestigt sein (nicht gezeigt).

Fig. 6 zeigt einen Torso mit befestigten EKG-Elektroden. Am Brustkorb (10) eines Patienten werden die EKG-Elektroden (8) angeordnet und mittels mehrmaligen Umwickelns mit Bündelstretchfolie (5) befestigt. In der Abbildung ist eine Anordnung der Brustwandableitung einer 10-poligen EKG-Ableitung gezeigt. Äquivalent zu den Brustwandelektroden werden die 4 weiteren Elektroden durch Umwickelung mit Bündelstretchfolie an den 4 Extremitäten (nicht gezeigt) befestigt.

## Patentansprüche

1. Befestigungsmittel für therapeutische und/oder diagnostische Medien, wie zum Beispiel therapeutische und/oder diagnostische Elektroden, Kälte- beziehungsweise Wärmeträger, Moorpackungen und dergleichen, an Extremitäten oder Körperteilen von Tieren oder Menschen, **dadurch gekennzeichnet, dass** die Befestigungsmittel (11) ein oder mehrere Streifen einer Stretchfolie (5) sind.

2. Befestigungsmittel nach Anspruch 1, **dadurch gekennzeichnet, dass** die Stretchfolie (5) im Wesentlichen aus Polyethylen besteht.

3. Befestigungsmittel nach Anspruch 1 oder 2, **dadurch gekennzeichnet, dass** die Stretchfolie (5) eine Breite zwischen 5 cm und 20 cm, bevorzugt etwa 10 cm hat.

4. Befestigungsmittel nach einem der Ansprüche 1 bis 3, **dadurch gekennzeichnet, dass** die Stretchfolie (5) eine Dehnspannung beim Zerreißen nach ISO 527-3 Norm von 250% bis 550% aufweist.

5. Befestigungsmittel nach einem der Ansprüche 1 bis 4, **dadurch gekennzeichnet, dass** die Stretchfolie (5) einen Reibungskoeffizienten, bevorzugt von etwa 0,8 aufweist, sodass aufeinanderliegende Folienabschnitte ohne Haftmittel aufeinander haften.

6. Befestigungsmittel nach einem der Ansprüche 1 bis 5, **dadurch gekennzeichnet, dass** die Stretchfolie (5) eine Dicke von 10 µm bis 60 µm aufweist.

7. Verfahren zur Befestigung von therapeutischen und/oder diagnostischen Medien, wie zum Beispiel therapeutische und/oder diagnostische Elektroden, Kälte- beziehungsweise Wärmeträger, Moorpackungen und dergleichen, an Extremitäten oder Körperteilen von Tieren oder Menschen, **dadurch gekennzeichnet, dass** das Medium an der Extremität (1) oder dem Körperteil (1,10) von Tier oder Mensch aufgelegt und durch zumindest einmalige Umwickelung mit Stretchfolie (5) befestigt wird, wobei gegebenenfalls zur Entfernung der Befestigung nach durchgeführter Therapie und/oder Diagnose die Stretchfolie (5) durch Zerschneiden, anderwärtiges Durchtrennen oder Abwickeln entfernt wird.
